# EUROPÄISCHE PATENTSCHRIFT

(11) **EP 0 853 808 B1**
(45) Veröffentlichungstag und Bekanntmachung des Hinweises auf die Patenterteilung: **11.08.1999**
(21) Anmeldenummer: 96945479.2
(22) Anmeldetag: 01.10.1996
(51) Int. Cl.: G21K 1/04

(54) **KONTURENKOLLIMATOR FÜR DIE STRAHLENTHERAPIE**
CONTOUR COLLIMATOR FOR RADIOTHERAPY
COLLIMATEUR PROFILE POUR RADIOTHERAPIE

(30) Priorität: 02.10.1995 DE 19536804
(43) Veröffentlichungstag der Anmeldung: 22.07.1998
(73) Patentinhaber: Deutsches Krebsforschungszentrum Stiftung des öffentlichen Rechts, 69120 Heidelberg (DE)
(72) Erfinder: PASTYR, Otto, D-69181 Leimen (DE); SCHLEGEL, Wolfgang, D-69121 Heidelberg (DE); HÖVER, Karl-Heinz, D-74889 Sinsheim (DE); MAIER-BORST, Wolfgang, D-69221 Dossenheim (DE)
(74) Vertreter: Castell, Klaus, Dr.-Ing.
(86) Internationale Anmeldenummer: DE9601892
(87) Internationale Veröffentlichungsnummer: WO9713255

(56) Entgegenhaltungen:
- EP-A- 0 387 921
- WO-A-94/29882
- FR-A- 2 485 790
- US-A- 4 313 349
- US-A- 5 555 283

## Beschreibung

Die Erfindung betrifft einen Konturenkollimator für die Strahlentherapie mit einer Mehrzahl von in einem Führungsblock vorgesehenen, relativ zueinander verschiebbar angeordneten plattenförmigen Blendenelementen zur Bildung einer konturierten Blende für ein von einer Strahlenquelle ausgehendes, auf den Kollimator gerichtetes Strahlenbündel und mit zumindest einem Antriebsorgan zur Verschiebung der Blendenelemente.

Ein derartiger Konturenkollimator ist aus der EP-A-0 387 921 B1 bekannt. Derartige Konturenkollimatoren dienen in der Strahlentherapie dazu, eine Blende zu formen, deren Öffnung der Kontur des zu bestrahlenden Bereiches im menschlichen Körper entspricht, so daß die von der Strahlenquelle ausgehenden hochenergetischen Strahlen nur diesen Bereich treffen und die Umgebung dieses Bereiches gegenüber der Strahlung abgeschirmt wird.

Bei dem bekannten Konturenkollimator ist für jeweils eine Gruppe einer vorgegebenen Anzahl von plattenförmigen Blendenelementen ein gemeinsames Verstellorgan vorgesehen, welches zum seriellen Verschieben jeweils eines ausgewählten Blendenelementes relativ zu den verbleibenden Blendenelementen dient. Hierzu steht ein Zahnrad des Verstellorgans mit einer am Blendenelement vorgesehenen Zahnstange im Eingriff und ein nicht drehbarer, verzahnter Bereich des Verstellorgans steht mit den restlichen Blendenelementen im Eingriff, um diese zu fixieren. Zur Beschleunigung des Einstellvorgangs wird beim Stand der Technik vorgeschlagen, auf jeder Seite des Konturenkollimators zwei derartige Verstellorgane vorzusehen.

Zur Verstellung der einzelnen Blendenelemente ist es beim Stand der Technik erforderlich, daß das jeweilige Verstellorgan zunächst translatorisch quer zu den Blendenelementen verschoben wird, damit das Verstellzahnrad mit der Zahnstange eines ausgewählten Blendenelements in Eingriff gerät. Dann wird das Zahnrad rotationsbeaufschlagt, um das zugeordnete Blendenelement zu verschieben. Dieser Vorgang muß für jedes Blendenelement einer Gruppe wiederholt werden.

Ein weiterer gattungsgemäßer Konturenkollimator ist aus der FR-A-2 485 790 bekannt. Bei diesem Konturenkollimator ist jedem Blendenelement ein Antriebsorgan zugeordnet. Die Antriebsorgane einer Gruppe von Blendenelementen sind benachbart angeordnet und zwischen jedem Antriebsorgan und dem zugeordneten Blendenelement ist eine Antriebsübertragungseinrichtung vorgesehen. Zur Verstellung der Blendenelemente wird ein linear wirkender elektrischer Motor verwendet.

Es ist die Aufgabe der vorliegenden Erfindung, einen gattungsgemäßen Konturenkollimator zu schaffen, der schneller einstellbar ist und der insgesamt einen einfacheren und damit wartungsärmeren und betriebssichereren Aufbau aufweist.

Diese Aufgabe wird gemäß dem kennzeichnenden Teil des Anspruchs 1 dadurch gelöst, daß jedem Blendenelement ein eigenes Antriebsorgan zugeordnet ist, daß die Antriebsorgane einer Gruppe von Blendenelementen im wesentlichen einander benachbart angeordnet sind und daß zwischen jedem Antriebsorgan und dem zugeordneten Blendenelement eine eigene Antriebsübertragungseinrichtung vorgesehen ist.

Dieser Aufbau gestattet es, trotz des sehr engen seitlichen Abstandes zwischen den einzelnen Blendenelementen, der in etwa der Dicke einer Blende, beispielsweise 1 mm, entspricht, jedes Blendenelement mit einem eigenen Antriebsorgan zu versehen und somit einzeln zu betätigen. Hierdurch wird die Einstellzeit für einen Konturenkollimator wesentlich beschleunigt, so daß einerseits die Bestrahlungszeit für einen jeden Patienten verkürzt wird, was für den Patienten eine Erleichterung darstellt und was auch gleichzeitig eine Erhöhung der Wirtschaftlichkeit mit sich bringt.

In einer vorteilhaften Ausführungsform sind die Antriebsorgane im wesentlichen halbkreisförmig angeordnet. Hierdurch wird ein besonders einfacher und übersichtlicher Aufbau erzielt, bei dem die Antriebsübertragungseinrichtungen im wesentlichen gleich lang sind, so daß für den Aufbau Gleichteile verwendet werden können.

Bei einer weiteren vorteilhaften Ausführungsform weist jede Antriebsübertragungseinrichtung ein flexibles, aber zug- und drucksteifes Kraftübertragungselement auf, das an seinem einen Ende mit dem zugeordneten Blendenelement und an seinem anderen Ende mit dem zugeordneten Antriebsorgan verbunden ist und das in einer Schubführung translatorisch verschiebbar gelagert ist. Ein derartiges Kraftübertragungselement gestattet eine besonders flexible Anordnung der Antriebsorgane.

Ist jedes Kraftübertragungselement über eine Kupplungsverbindung mit dem ihm zugeordneten Blendenelement lösbar gekuppelt, so wird hierdurch ein einfacher Aufbau des Konturenkollimators geschaffen, der zudem ein schnelles und problemloses Auswechseln einzelner Elemente gestattet.

Der gleiche Vorteil tritt auf, wenn jedes Kraftübertragungselement über eine Kupplungsverbindung mit dem ihm zugeordneten Antriebsorgan lösbar gekuppelt ist.

Vorteilhafterweise weist jedes Kraftübertragungselement ein Federband auf.

Jedes Antriebsorgan ist bevorzugt von einem linear wirkenden Motor gebildet. Hierdurch wird ein besonders schlanker Aufbau der Anordnung von Antriebsorganen ermöglicht, so daß die Anordnung von Antriebsorganen sehr kompakt sein kann.

Dabei ist der Motor bevorzugt ein elektrischer Linearmotor.

Alternativ dazu ist der Motor ein Elektromotor mit einem linear wirkenden Getriebe, vorzugsweise einem Zahnstangengetriebe oder einem Spindelgetriebe.

Weist der Führungsblock eine obere und eine untere Führungsplatte auf, die jeweils mit einer Mehrzahl von oberen beziehungsweise unteren Führungsrillen für die Blendenelemente versehen sind, so wird eine besonders zuverlässige und funktionssichere Verstellbarkeit der Blendenelemente gewährleistet.

In einer bevorzugten Ausbildung sind die obere und die untere Führungsplatte jeweils mit einer vorzugsweise rechteckigen Öffnung versehen, die die maximale Blendenöffnung bestimmen und eine gemeinsame, zur Längsrichtung der Führungsrillen im wesentlichen rechtwinklig verlaufende Mittelebene aufweisen.

Sind die Schubführungen im wesentlichen nebeneinander in einem Schubführungsblock angeordnet und weisen sie fächerartig gekrümmt auseinanderlaufende Schubführungsspalten auf, in denen jeweils ein Kraftübertragungselement translatorisch verschiebbar aufgenommen ist, so wird eine sichere Führung der Kraft-übertragungselemente erzielt, so daß eine genaue translatorische Verstellung der Blendenelemente möglich ist, da ein unerwünschtes Ausbeulen der Kraftübertragungselemente durch die eng am jeweiligen Kraftübertragungselement anliegenden Spaltenwände verhindert wird.

Eine besonders kompakte Anordnung wird dann gebildet, wenn jedem Schubführungsblock zwei übereinandergelegene Ebenen von Anriebsorgan-Anordnungen zugeordnet sind, wobei zwei übereinandergelegene Antriebsorgane jeweils ein in nebeneinandergelegenen Schubführungen aufgenommenes Kraftübertragungselement beaufschlagen. Hierdurch kann die Gesamtbreite des Konturenkollimators trotz einer Vielzahl von verstellbaren Blendenelementen wirksam begrenzt werden.

Sind im Führungsblock zwei einander gegenübergelegene Gruppen von translatorisch antreibbaren Blendenelementen vorgesehen, wobei jeweils zwei einander gegenübergelegene Blendenelemente in einer unteren und einer oberen gemeinsamen Führungsrille geführt sind, so wird einerseits durch das Vorsehen der einander gegenübergelegenen Gruppen von Blendenelementen die Möglichkeit geschaffen, über einen Winkel von 360° umlaufende Konturen einzustellen und andererseits ermöglicht, durch Berührung zweier gegenübergelegener Blendenelemente eine vollständige Abschirmung im Bereich der betreffenden Führungsrille zu erzielen.

Ist jedes Blendenelement eines Paares von einander gegenübergelegenen Blendenelementen mit seiner freien, vom jeweiligen Antriebsorgan abgewandten Kante bis über die gemeinsame Mittelebene der Öffnungen in der unteren und der oberen Führungsplatte hinaus verschiebbar, so können Konturen erzeugt werden, die starke einseitige Einschnürungen aufweisen, wie dies beispielsweise bei nierenförmigen Konturen der Fall ist.

Bevorzugt ist jedem Antriebsorgan ein Wegaufnehmer, vorzugsweise ein Potentiometer, zur Erfassung der aktuellen Position des entsprechenden Blendenelements zugeordnet. Hierdurch wird eine genaue Steuerung der Blendenelementen-Positionen ermöglicht, so daß beispielsweise die Einstellung der Kontur über ein Computerprogramm automatisch erfolgen kann.

Besonders zuverlässig und kostengünstig ist diese Ausgestaltung dann, wenn der Wegaufnehmer ein translatorisch betätigbares Schiebepotentiometer aufweist.

Ist zumindest eines der im Bereich des zentralen Mittelstrahls des Strahlenbündels gelegenen Blendenelemente mit wenigstens einer in Translationsrichtung verlaufenden Verdickungsrippe versehen, so wird eine zuverlässige Abschattung des zentralen Mittelstrahls erreicht, da die Verdickungsrippe den parallel zum Blendenelement verlaufenden Mittelstrahl abschattet. Alternativ können auch die Blendenelemente zum Strahl hin schräg gestellt werden. Ferner kann alternativ ein mittleres Blendenelement oben dicker als unten ausgebildet werden.

Diese Abschattungswirkung wird dann noch verstärkt, wenn jede Verdickungsrippe in eine entsprechende Nut im benachbarten Blendenelement eingreift.

Die Erfindung wird nachfolgend anhand eines Beispiels unter Bezugnahme auf die Zeichnung näher erläutert; in dieser zeigt:
Fig. 1 eine perspektivische Draufsicht auf einen erfindungsgemäßen Konturenkollimator;
Fig. 2 A eine weggebrochene Teilansicht eines Führungsblocks mit einigen eingesetzten Blendenelementen;
Fig. 2 B eine Seitenansicht eines Führungsblocks in Translationsrichtung der Blendenelemente;
Fig. 2 C einen Ausschnitt aus Fig. 2 B, der die in die Führungsschienen eingesetzten Blendenelemente im Bereich der unteren Führungsplatte zeigt;
Fig. 3 eine perspektivische Draufsicht auf eine zweite Ausführungsform eines Konturenkollimators, der mit Wegaufnehmern versehen ist;
Fig. 4 eine Schnittansicht durch einen Konturenkollimator entsprechend der durch strichpunktierte Linien angegebenen Längsmittelebene IV-IV in Fig. 3 in einer ersten Stellung der Blendenelemente;
Fig. 5 eine dem Schnitt in Fig. 4 entsprechende Schnittdarstellung in einer Kalibrierstellung der Blendenelemente;
Fig. 6 eine Montageansicht eines Kraftübertragungselements an einem Blendenelement;
Fig. 7 unterschiedliche Blendenelemente mit und ohne Verdickungsrippen und
Fig. 8 eine schematische Darstellung der Strahlenabschattung bei einem erfindungsgemäßen Konturenkollimator.

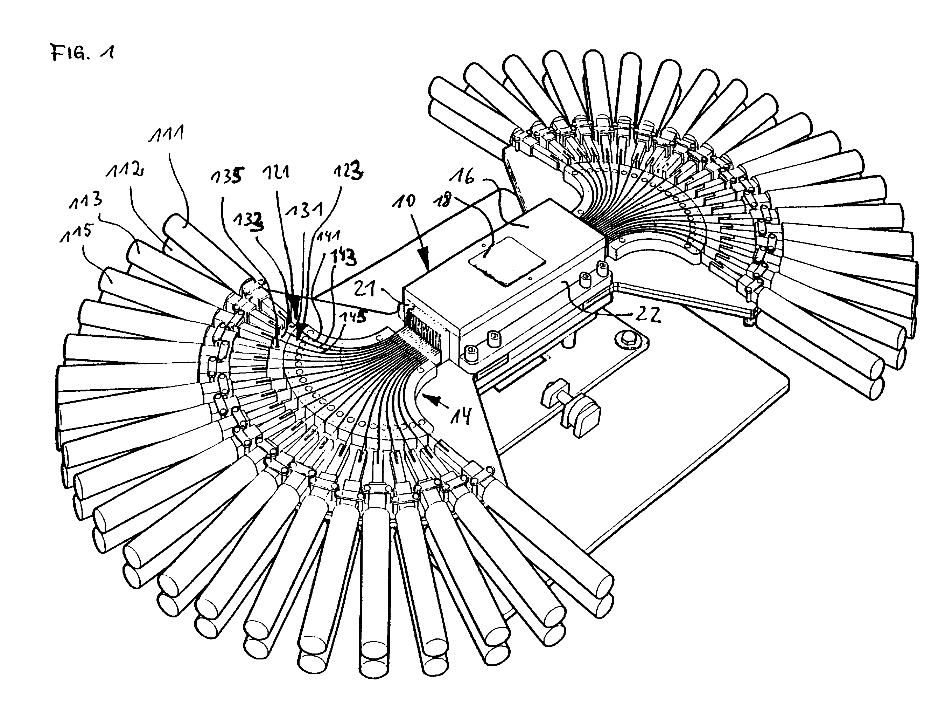

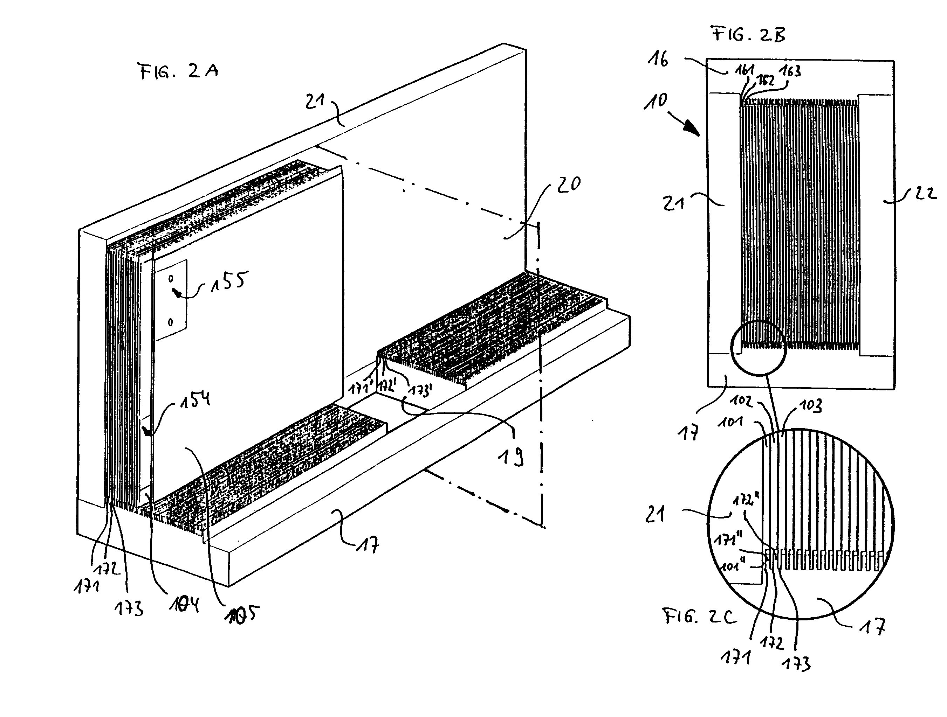

In Fig. 1 ist eine perspektivische Ansicht eines erfindungsgemäßen Konturenkollimators gezeigt, dessen Herzstück von einem Führungsblock 10 gebildet ist, der im Detail in den Fig. 2 A bis 2 C dargestellt ist.

Der Führungsblock 10 weist eine untere Führungsplatte 17, eine obere Führungsplatte 16 sowie zwei Seitenwände 21 und 22 auf. In der oberen Führungsplatte 16 ist eine im wesentlichen rechteckige Öffnung 18 mittig vorgesehen. Eine im wesentlichen vertikal mit der oberen Öffnung 18 fluchtende untere Öffnung 19 ist in der unteren Führungsplatte 17 mittig vorgesehen. Die untere Führungsplatte 17 ist auf ihrer Oberseite mit einer Vielzahl von sich in Längsrichtung der unteren Führungsplatte 17 erstreckenden unteren Führungsrillen 171, 172, 173, ... versehen, die parallel zueinander in gleichem seitlichem Abstand und auf der einen Seite der unteren Öffnung 19 ausgebildet sind. Die obere und die untere Führungsplatte 16, 17 bestehen bevorzugt aus Messing, Bronze oder Keramik, oder einem strahlungsbeständigen Material mit guten Gleiteigenschaften.

Auf der anderen Seite bezüglich der unteren Öffnung 19 sind fluchtend mit den unteren Führungsrillen 171, 172, 173, ... weitere untere Führungsrillen 171', 172', 173', ... ausgebildet. Auf die gleiche Weise sind auf der Unterseite der oberen Führungsplatte 16 obere Führungsrillen 161, 162, 163, ... sowie nicht gezeigte weitere obere Führungsrillen, die mit den Führungsrillen 161, 162, 163, ... fluchten und auf der anderen Seite der oberen Öffnung 18 ausgebildet sind, vorgesehen.

Da der Führungsblock 10 bezüglich der rechtwinklig zu den Führungsrillen 161, 162, 163, ...; 171, 172, 173, ...; 171', 172', 173', ... durch die Mitte der Öffnungen 18 und 19 verlaufenden Mittelebene 20 symmetrisch ausgebildet ist, wird nachfolgend zur Vereinfachung lediglich der Aufbau des Führungsblocks auf einer Seite bezüglich der Mittelebene 20 beschrieben. Der Aufbau auf der anderen Seite ist dazu analog ausgebildet.

In jede Paarung der vertikal übereinandergelegenen Führungsrillen 161, 171; 162, 172; 163, 173; ... ist ein plattenartiges Blendenelement 101; 102; 103; ... translatorisch verschiebbar eingesetzt.

Wie aus Fig. 2 C zu erkennen ist, entspricht die Breite der einzelnen Führungsrillen 161, 171, ... etwa der Hälfte der Dicke eines Blendenelements 101, ..., wobei die Dicke eines plattenartigen Blendenelements etwa 1 mm beträgt. Zwischen zwei benachbarten Führungsrillen 171, 172 ist ein Steg 171" ausgebildet, dessen Breite etwas größer ist als die Breite der benachbarten Führungsrillen 171, 172 und damit auch etwas größer ist als die Hälfte der Breite eines Blendenelements. Jedes Blendenelement besitzt an seiner unteren Kante einen Abschnitt 101" von reduzierter Dicke, der in die zugehörige Führungsrille 171 eingesetzt und in dieser translatorisch verschiebbar ist. Ein analog ausgebildeter oberer Abschnitt verringerter Dicke, der in die Führungsrille 161 eingreift, ist zwar in der Zeichnung nicht dargestellt, doch sind sowohl die oberen Kanten der Blendenelemente 101, 102, 103, ... sowie die obere Führungsplatte im Bereich der oberen Führungsrillen 161, 162, 163, ... analog ausgebildet, wie die unteren Kanten der Blendenelemente 101, 102, 103, ... und die untere Führungsplatte 17 im Bereich ihrer Führungsrillen 171, 172, 173, ..., wie dies in Fig. 2 C dargestellt ist.

Aufgrund der unterschiedlichen Breiten der Führungsrillen 171, 172, 173 und der zwischen ihnen gelegenen Stege 171", 172" sind die in die Führungsrillen 171, 172 eingesetzten Blendenelemente 101, 102 geringfügig seitlich voneinander beabstandet, so daß sie einander nicht berühren.

An ihrer von der Mittelebene 20 abgewandten Vertikalkante sind die Blendenelemente mit Kupplungsverbindungen 154, 155 versehen, wie dies in Fig. 2 A anhand der Platten 174 und 175 zu erkennen ist. Die Kupplungsverbindungen 154, 155 sind abwechselnd in der Nähe der unteren Führungsplatte 17 und der oberen Führungsplatte 16 ausgebildet, so daß sie bei nebeneinandergelegenen Blendenelementen jeweils nach oben beziehungsweise nach unten versetzt sind.

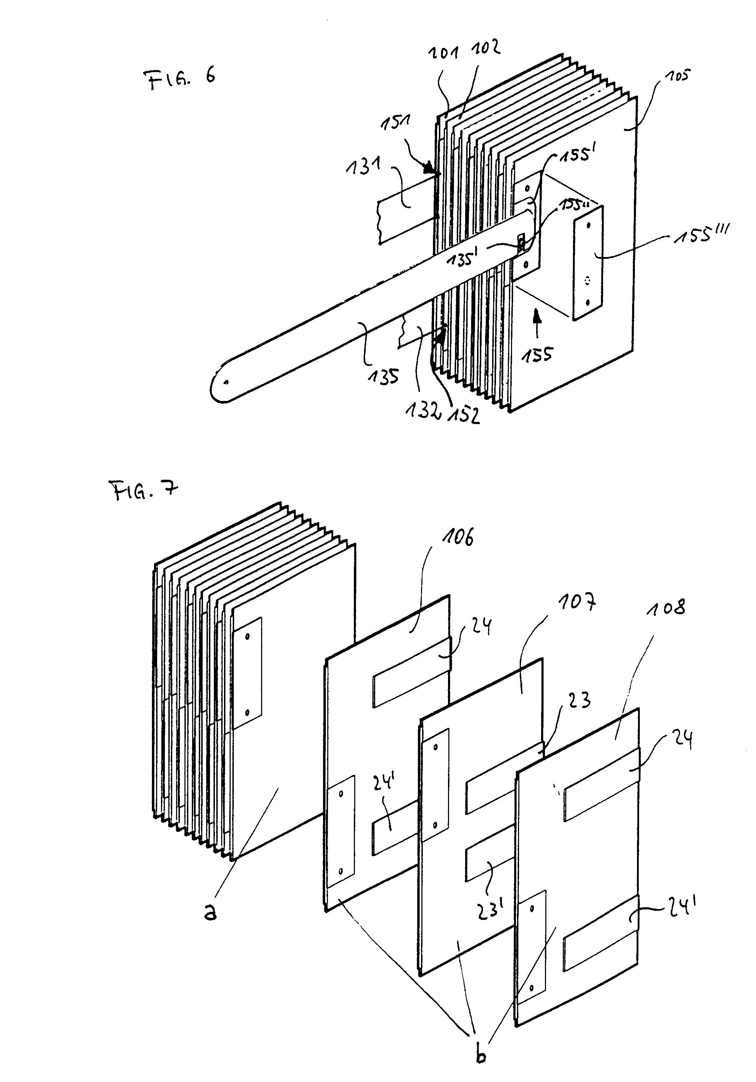

Eine derartige Kupplungsverbindung ist in Fig. 6 für das Blendenelement 105 detaillierter dargestellt. Die Kupplungsverbindung 155 umfaßt eine abgestufte Ausnehmung 155' in einer Seitenfläche des Blendenelements 105. Im tieferen Teil der abgestuften Ausnehmung 155' ist ein Zapfen 155" vorgesehen, der bei der Herstellung der Ausnehmung stehenbleibt und der der vollständigen Dicke des Blendenelements 105 entspricht. Diese Ausnehmung 155' wird durch Ausfräsen aus der Oberfläche des metallenen Blendenelements, welches vorzugsweise aus Wolfram besteht, hergestellt.

Die abgestufte Ausnehmung ist in ihrem tieferen Bereich von einer derartigen Abmessung, daß ein Kraftübertragungselement 135 mit einer hakenartigen Ausnehmung 135' in diesen tieferen Bereich einsetzbar ist und den Zapfen 155" hintergreift. Die Dicke des Kraftübertragungselements entspricht dabei ungefähr der Höhe der Stufe innerhalb der abgestuften Ausnehmung 155' und beträgt vorzugsweise 0,30 mm, während die Höhe des Kraftübertragungselements etwa 13 mm beträgt. Das Kraftübertragungselement 135 besteht vorzugsweise aus Federstahl.

Nachdem eine Abdeckplatte 155"' in den weniger stark vertieften Bereich der abgestuften Vertiefung 155' eingesetzt und dort verankert wurde, wird das Kraftübertragungselement 135 mit seiner hakenartigen Ausnehmung 135' in den tieferen Abschnitt der abgestuften Vertiefung 155' eingehängt, wobei dabei der Zapfen 155" hintergriffen wird. Dadurch wird ein seitliches Herausfallen des Kraftübertragungselements 135 verhindert. Auf diese Weise ist eine gelenkige Kupplungsverbindung 155 zwischen dem Blendenelement 105 und dem Kraftübertragungselement 135 gebildet.

Die aus Federstahl bestehenden, flexiblen, aber zug- und drucksteifen Kraftübertragungselemente 131, 132, 133, ..., 135, ... sind jeweils in einer oberen beziehungsweise unteren Schubführung aufgenommen, von denen in Fig. 1 lediglich die oberen Schubführungen 141, 143, 145 zu sehen sind. Die Schubführungen für die Kraftübertragungselemente, die den Blendenelementen 102, 104, ... zugeordnet sind, welche in ihrem unteren Bereich mit der Kupplungsverbindung versehen sind, sind unterhalb der in Fig. 1 dargestellten Schubführungen in einer daruntergelegenen Ebene vorgesehen.

Die Schubführungen 141, 143, 145, ... sind - ebenso wie jene der daruntergelegenen Ebene - fächerförmig nach außen gekrümmt angeordnet, wobei der Krümmungsradius der Schubführungen nach außen hin abnimmt, das heißt, daß der Krümmungsradius der Schubführung 141 für das Kraftübertragungselement 133 des näher zur Seitenwand 21 gelegenen Blendenelements 101 geringer ist, also die Krümmung größer ist, als jener Krümmungsradius für ein Kraftübertragungselement eines mehr in der Mitte gelegenen Blendenelements. Auf der anderen Seite, das heißt, zur anderen Seitenwand 22 hin, nimmt der Krümmungsradius der Schubführungen wieder ab, so daß dort die Krümmung wieder steigt. Auf diese Weise werden die Kraftübertragungselemente in einem Winkel von etwa 180° aufgefächert, wie in Fig. 1 zu sehen ist.

An ihrem aufgefächerten, freien Ende sind die Kraftübertragungselemente 131, 132, 133, ..., 135, ... mit jeweils einem als Linearantrieb ausgebildeten Antriebsorgan 111, 112, 113, ..., 115, ... gekoppelt. So sind durch diese Kopplung zwischen dem jeweiligen Antriebsorgan 111, 112, 113, ..., 115, ... mit dem Kraftübertragungselement 131, 132, 133, ..., 135, der Kupplungsverbindung 151, 152, ..., 155, ... mit dem jeweiligen Blendenelement 101, 102, ..., 105, ... Antriebsübertragungseinrichtungen 121, 122, ..., 125, ... zur Übertragung der vom jeweiligen Antriebsorgan erzeugten Linearbewegung auf das zugehörige Blendenelement geschaffen.

Die in Fig. 1 dargestellte Auffächerung der einzelnen Antriebsübertragungseinrichtungen 121, 122, ... gestattet auf besonders vorteilhafte Weise die Anordnung einer Vielzahl von Antriebsorganen 111, 113, 115, ... nebeneinander auf engstem Raum, wobei gleichzeitig die in Fig. 1 dargestellte Anordnung der Antriebsorgane in zwei übereinandergelegenen Ebenen, wie dies anhand der Antriebsorgane 111 und 112 dargestellt ist, nochmals die Kompaktheit erhöht.

Bei dem in Fig. 1 gezeigten Beispiel sind demnach, wenn man die nebeneinandergelegenen Blendenelemente numeriert, die ungeradzahligen Blendenelemente in ihrem oberen Bereich mit der Kupplungsverbindung versehen, in welcher das jeweilige Kraftübertragungselement angelenkt ist, welches durch eine obere fächerartige Anordnung von Schubführungen zu einer oberen Reihe von Antriebsorganen führt, während die geradzahligen Blendenelemente in ihrem unteren Bereich die Kupplungsverbindung aufweisen, die sie mit den Kraftübertragungselementen gelenkig verbindet, welche durch eine untere fächerartige Anordnung von Schubführungen zu einer unteren Reihe von Antriebsorganen verläuft. Durch diese Anordnung ist es möglich, besonders viele äußerst schmale Blendenelemente dicht nebeneinander anzubringen und jeweils mit einem eigenen Antriebsorgan zu beaufschlagen.

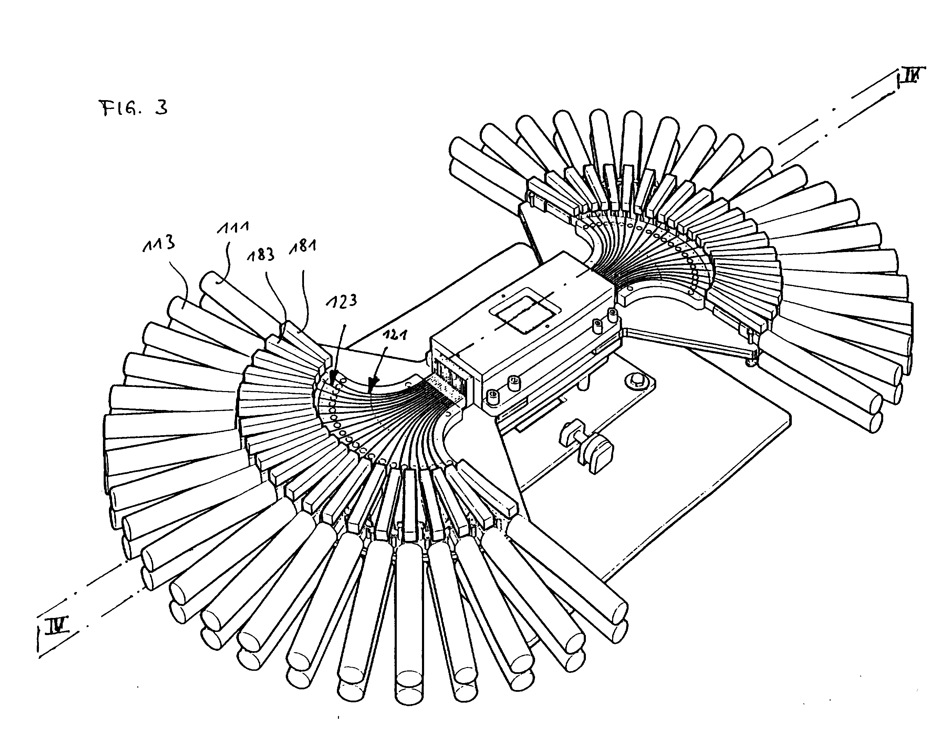

Eine alternative Ausgestaltung der in Fig. 1 gezeigten Anordnung ist in Fig. 3 dargestellt, wobei der Übersichtlichkeit halber nur wenige Bezugszeichen eingetragen sind. Jeder Antriebsübertragungseinrichtung 121, 123 ist im Bereich des zugehörigen Antriebsorgans 111, 113 ein Wegaufnehmer 181, 183 zugeordnet, der den Grad der translatorischen Verschiebung mißt und an eine Steuerschaltung weitergibt. Über diese Wegaufnehmer 181, 183, ..., die vorzugsweise von Schiebepotentiometern gebildet sind, läßt sich die Lage jedes einzelnen Blendenelements 101, 103, ... feststellen, so daß eine computergesteuerte genaue Kontur im Kollimator eingestellt werden kann.

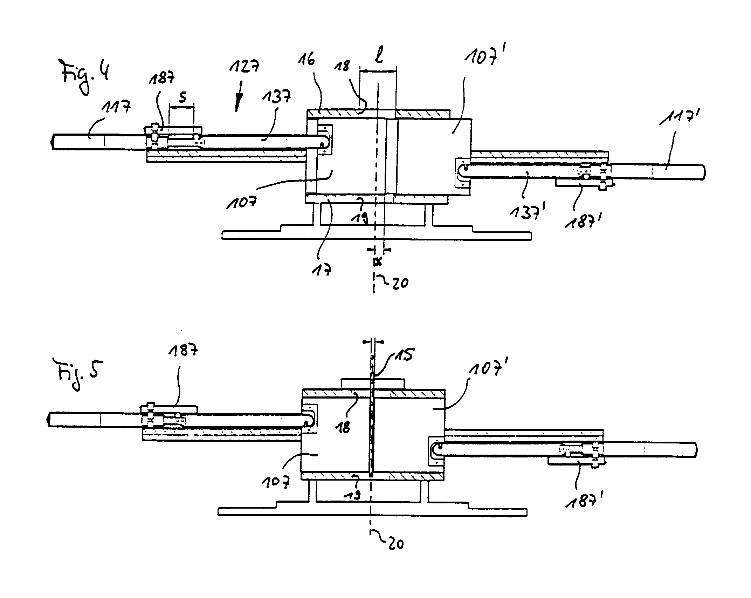

Fig. 4 zeigt einen erfindungsgemäßen Konturenkollimator im Längsschnitt, wobei ein vorderes Blendenelement 107 einer ersten Gruppe von Blendenelementen, angetrieben von einem Antriebsorgan 117 über die das Kraftübertragungselement 137 aufweisende Antriebsübertragungseinrichtung 127 in eine Position verfahren ist, in welcher die im Bereich der Öffnungen 18 und 19 in der oberen Führungsplatte beziehungsweise der unteren Führungsplatte 17 gelegene freie Kante des Blendenelements 107 über die Mittelebene 20 hinaus steht, so daß das Blendenelement 107 mehr als 50 % der Längserstreckung der Öffnung 18 beziehungsweise 19 abschattet. Das gegenübergelegene Blendenelement 107' einer zweiten Gruppe ist von seinem Antriebsorgan 117' über sein Kraftübertragungselement 137' soweit zurückgezogen worden, daß seine den Öffnungen 18, 19 zugewandte freie Kante aus dem Querschnitt der Öffnungen 18 beziehungsweise 19 herausgetreten ist.

Die mögliche Verfahrstrecke s der Antriebsübertragungseinrichtung 127 ist somit, wie beim zugehörigen Wegaufnehmer 187 dargestellt, um den Betrag x größer als die Längserstreckung 1 der Öffnungen 18 beziehungsweise 19. Hierdurch wird es möglich, mit dem erfindungsgemäßen Konturenkollimator Konturen zu erzeugen, die starke einseitige Einschnitte aufweisen, wie dies beispielsweise bei nierenförmigen Konturen der Fall ist.

In Fig. 5 ist eine Position der Blendenelemente 107, 107' gezeigt, in welcher die jeweilige im Bereich der Öffnungen 18, 19 gelegene freie Kante der Blendenelemente 107, 107' gegen eine die Öffnungen 18, 19 vertikal durchgreifende und zentriert in der Mittelebene 20 fixierte Kalibierplatte 15 anliegen. In dieser Stellung sind beide Blendenelemente 107, 107' bezüglich der Mittelebene 20 symmetrisch gelegen, so daß in der in Fig. 5 gezeigten Einstellung entweder die zugehörigen Wegaufnehmer 187, 187' symmetrisch positioniert werden können oder die von diesen Wegaufnehmern 187, 187' gelieferten Signale in einer Steuereinheit als Symmetrie-Referenzsignale gespeichert werden können.

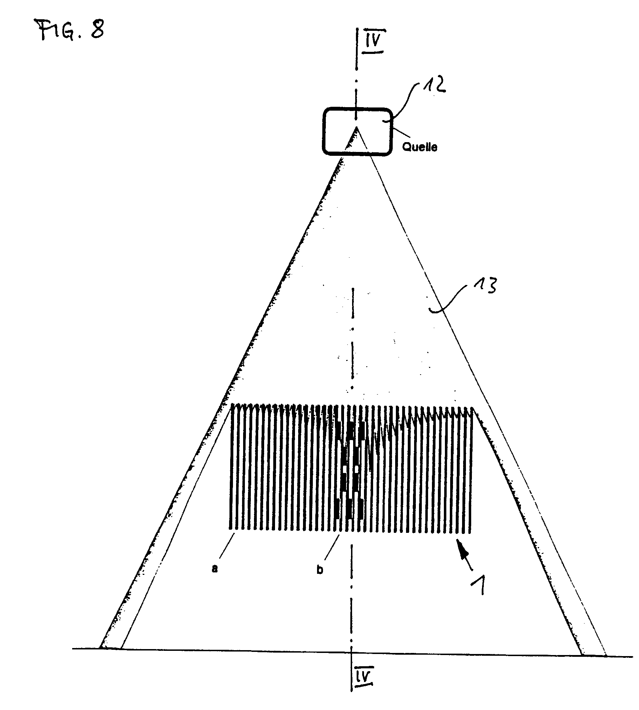

In Fig. 7 sind unterschiedliche Arten von Blendenelementen dargestellt, wobei die mit a bezeichneten Blendenelemente jenen (101, 102, ..., 105) entsprechen, die vorstehend bereits abgehandelt und beschrieben worden sind. Die mit b bezeichneten Blendenelemente 106, 107, 108 sind für den Einsatz im Bereich der Längsmittelebene des Konturenkollimators ausgebildet, die in Fig. 3 mit IV-IV bezeichnet ist. In dieser Längsmittelebene ist, wie in Fig. 8 dargestellt ist, üblicherweise auch das Zentrum der Strahlenquelle 12 gelegen, von der das Strahlenbündel 13 kegelförmig nach unten auf den Kollimator 1 gerichtet ist.

Wären im Bereich der Längsmittelebene IV-IV Blendenelemente des Typs a angeordnet, so würde der von der Strahlenquelle 12 ausgehende Mittelstrahl und die ihm unmittelbar benachbarten Strahlen nahezu ungehindert und nicht abgeschattet durch die Zwischenräume der Blendenelemente hindurchgehen, so daß der Kollimator in der Nähe der Längsmittelebene IV-IV wirkungslos wäre. Aus diesem Grund sind, wie in Fig. 7 dargestellt ist, die im Bereich der Längsmittelebene IV-IV angeordneten Blendenelemente des Typs b mit Verdickungsrippen 23, 23' beziehungsweise 24, 24' versehen. Die Verdickungsrippen verlaufen dabei in Richtung der Translationsbewegung der jeweiligen Blendenelemente und erstrecken sich über die gesamte Länge oder zumindest über mehr als 50 % der Länge des jeweiligen Blendenelements in Translationsrichtung.

Die jeweiligen Verdickungsrippen 23, 23'; 24, 24' greifen in (nicht gezeigte) entsprechend ausgebildete Nuten auf der gegenübergelegenen Seite des benachbarten Blendenelementes ein, so daß der Spalt zwischen zwei benachbarten Blendenelementen von den jeweiligen Verdickungselementen strahlungsdicht unterbrochen ist. Auf diese Weise ist das Hindurchtreten des Mittelstrahls beziehungsweise der ihm benachbarten Strahlen durch die zwischen den Blendenelementen des Typs b gebildeten Spalte wirksam verhindert, wie in Fig. 8 dargestellt ist.

Um eine Materialschwächung der benachbarten Blendenelemente des Typs b im Bereich der Nuten und der Verdickungen zu verhindern, sind die Verdickungen 23, 23' beziehungsweise 24, 24' zweier benachbarter Blendenelemente des Typs b sowie die in ihnen vorgesehenen zugeordneten Nuten über die Höhe des jeweiligen Blendenelements versetzt angeordnet, wie in Fig. 7 dargestellt ist.

### Bezugszeichenliste

- 1: Konturenkollimator
- 10: Führungsblock
- 12: Strahlenquelle
- 13: Strahlenbündel
- 14: Schubführungsblock
- 15: Kalibrierplatte
- 16: Obere Führungsplatte
- 17: Untere Führungsplatte
- 18: Obere Öffnung
- 19: Untere Öffnung
- 20: Mittelebene
- 21: Seitenwand
- 22: Seitenwand
- 23: Verdickungsrippe
- 23': Verdickungsrippe
- 24: Verdickungsrippe
- 24': Verdickungsrippe
- 101: Blendenelement
- 101": Abschnitt von reduzierter Dicke
- 102: Blendenelement
- 103: Blendenelement
- 104: Blendenelement
- 105: Blendenelement
- 106: Blendenelement
- 107: Blendenelement
- 107': Blendenelement
- 108: Blendenelement
- 111: Antriebsorgan
- 112: Antriebsorgan
- 113: Antriebsorgan
- 115: Antriebsorgan
- 117: Antriebsorgan
- 117': Antriebsorgan
- 121: Antriebsübertragungseinrichtung
- 123: Antriebsübertragungseinrichtung
- 127: Antriebsübertragungseinrichtung
- 131: Kraftübertragungselement
- 132: Kraftübertragungselement
- 133: Kraftübertragungselement
- 135: Kraftübertragungselement
- 135': Hakenartige Ausnehmung
- 137: Kraftübertragungselement
- 137': Kraftübertragungselement
- 141: Schubführung
- 143: Schubführung
- 145: Schubführung
- 151: Kupplungsverbindung
- 152: Kupplungsverbindung
- 155: Kupplungsverbindung
- 155': Abgestufte Ausnehmung
- 155": Zapfen
- 155"': Abdeckplatte
- 161: Obere Führungsrille
- 162: Obere Führungsrille
- 163: Obere Führungsrille
- 171: Untere Führungsrille
- 171': Untere Führungsrille
- 171": Steg
- 172: Untere Führungsrille
- 172': Untere Führungsrille
- 172": Steg
- 173: Untere Führungsrille
- 173': Untere Führungsrille
- 181: Wegaufnehmer
- 183: Wegaufnehmer
- 187: Wegaufnehmer

## Patentansprüche

1. Konturenkollimator (1) für die Strahlentherapie mit einer Mehrzahl von in einem Führungsblock (10) vorgesehenen, relativ zueinander verschiebbar angeordneten plattenförmigen Blendenelementen (101, 102, 103,...) zur Bildung einer konturierten Blende für ein von einer Strahlenquelle (12) ausgehendes, auf den Kollimator gerichtetes Strahlenbündel (13) und mit zumindest einem Antriebsorgan zur Verschiebung der Blendenelemente, dadurch gekennzeichnet,
daß jedem Blendenelement (101, 102, 103,...) ein eigenes Antriebsorgan (111, 112, 113, ...) zugeordnet ist,
daß die Antriebsorgane (111, 112, 113, ...) einer Gruppe von Blendenelementen (101, 102, 103,...) im wesentlichen einander benachbart angeordnet sind und
daß zwischen jedem Antriebsorgan (111, 112, 113, ...) und dem zugeordneten Blendenelement (101, 102, 103,...) eine eigene Antriebsübertragungseinrichtung (121, 122, 123,...) vorgesehen ist.

2. Konturenkollimator nach Anspruch 1,
dadurch gekennzeichnet,
daß die Antriebsorgane (111, 112, 113, ...) im wesentlichen halbkreisförmig angeordnet sind.

3. Konturenkollimator nach Anspruch 1 oder 2,
dadurch gekennzeichnet,
daß jede Antriebsübertragungseinrichtung (121, 122, 123,...) ein flexibles, aber zug- und drucksteifes Kraftübertragungselement (131, 132, 133,...) aufweist, das an seinem einen Ende mit dem zugeordneten Blendenelement (101, 102, 103,...) und an seinem anderen Ende mit dem zugeordneten Antriebsorgan (111, 112, 113, ...) verbunden ist und das in einer Schubführung (141, 142, 143,...) transiatorisch verschiebbar gelagert ist.

4. Konturenkollimator nach Anspruch 3,
dadurch gekennzeichnet,
daß jedes Kraftübertragungselement (131, 132, 133,...) über eine Kupplungsverbindung (151, 152, 153,...) mit dem ihm zugeordneten Blendenelement (101, 102, 103,...) lösbar gekuppelt ist.

5. Konturenkollimator nach Anspruch 3 oder 4,
dadurch gekennzeichnet,
daß jedes Kraftübertragungselement (131, 132, 133,...) über eine weitere Kupplungsverbindung mit dem ihm zugeordneten Antriebsorgan (111, 112, 113, ...) lösbar gekuppelt ist.

6. Konturenkollimator nach einem der Ansprüche 3 bis 5,
dadurch gekennzeichnet,
daß jedes Kraftübertragungselement (131, 132, 133,...) ein Federband aufweist.

7. Konturenkollimator nach einem der vorhergehenden Ansprüche,
dadurch gekennzeichnet,
daß jedes Antriebsorgan (111, 112, 113, ...) von einem linear wirkenden Motor gebildet ist.

8. Konturenkollimator nach Anspruch 7,
dadurch gekennzeichnet,
daß der Motor (111, 112, 113, ...) ein elektrischer Linearmotor ist.

9. Konturenkollimator nach Anspruch 7,
dadurch gekennzeichnet,
daß der Motor (111, 112, 113, ...) ein Elektromotor mit einem linear wirkenden Getriebe, vorzugsweise einem Zahnstangengetriebe oder einem Spindelgetriebe, ist.

10. Konturenkollimator nach einem der vorhergehenden Ansprüche,
dadurch gekennzeichnet,
daß der Führungsblock (10) eine obere (16) und eine untere Führungsplatte (17) aufweist, die jeweils mit einer Mehrzahl von oberen (161, 162, 163,...) bzw. unteren Führungsrillen (171, 172, 173,...) für die Blendenelemente (101, 102, 103,...) versehen sind.

11. Konturenkollimator nach Anspruch 10,
dadurch gekennzeichnet,
daß die obere (16) und die untere Führungsplatte (17) jeweils mit einer vorzugsweise rechteckigen Öffnung (18, 19) versehen sind, die die maximale Blendenöffnung bestimmen und eine gemeinsame, zur Längsrichtung der Führungsrillen (161, 162, 163, ...; 171, 172, 173,...) im wesentlichen rechtwinklig verlaufende Mittelebene (20) aufweisen.

12. Konturenkollimator nach einem der Ansprüche 3 bis 11,
dadurch gekennzeichnet,
daß die Schubführungen (141, 142, 143,...) im wesentlichen nebeneinander in einem Schubführungsblock (14) angeordnet sind und fächerartig gekrümmt auseinanderlaufenden Schubführungsspalten aufweisen, in denen jeweils ein Kraftübertragungselement (131, 132, 133,...) translatorisch verschiebbar aufgenommen ist.

13. Konturenkollimator nach einem der vorhergehenden Ansprüche,
dadurch gekennzeichnet,
daß jedem Schubführungsblock (14) zwei übereinander gelegene Ebenen von Antriebsorgan-Anordnungen zugeordnet sind, wobei zwei übereinander gelegene Antriebsorgane (111, 112, 113, ...) jeweils ein in nebeneinander gelegenen Schubführungen (141, 142, 143,...) aufgenommenes Kraftübertragungselement (131, 132, 133, ...) beaufschlagen.

14. Konturenkollimator nach einem der vorhergehenden Ansprüche,
dadurch gekennzeichnet,
daß im Führungsblock (10) zwei einander gegenübergelegene Gruppen von translatorisch antreibbaren Blendenelementen (101, 102, 103,...;101', 102', 103',...) vorgesehen sind, wobei jeweils zwei einander gegenübergelegene Blendenelemente (101, 101'; 102, 102'; 103, 103'; ...) in einer unteren (161, 161'; 162, 162'; 163, 163';...) und einer oberen (171, 171'; 172, 172'; 173, 173';...) gemeinsamen Führungsrille geführt sind.

15. Konturenkollimator nach einem der vorhergehenden Ansprüche,
dadurch gekennzeichnet,
daß jedes Blendenelement (101, 101', 102, 102', 103, 103', ...) eines Paares von einander gegenübergelegenen Blendenelementen mit seiner freien, vom jeweiligen Antriebsorgan (111, 111', 112, 112', 113, 113', ...) abgewandten Kante bis über die gemeinsame Mittelebene (20) der Öffnungen (18, 19) in der oberen (16) und der unteren (17) Führungsplatte hinaus verschiebbar ist.

16. Konturenkollimator nach einem der vorhergehenden Ansprüche,
dadurch gekennzeichnet,
daß daß jedem Antriebsorgan (111, 112, 113, ...) mindestens ein Wegaufnehmer (181, 182, 183, ...), vorzugsweise ein Potentiometer, zum Erfassen der Position des entsprechenden Blendenelements (101, 102, 103,...) zugeordnet ist.

17. Konturenkollimator nach Anspruch 16,
dadurch gekennzeichnet,
daß der Wegaufnehmer (181, 182, 183, ...) ein translatorisch betätigbares Schiebepotentiometer aufweist.

18. Konturenkollimator nach einem der vorhergehenden Ansprüche,
dadurch gekennzeichnet,
daß zumindest eines der im Bereich des zentralen Mittelstrahls des Strahlenbündels (13) gelegenen Blendenelemente (106, 107, 108) mit wenigstens einer in Translationsrichtung verlaufenden Verdickungsrippe (23, 23', 24, 24') versehen ist.

19. Konturenkollimator nach Anspruch 18,
dadurch gekennzeichnet,
daß jede Verdickungsrippe (23, 23'; 24, 24') in eine entsprechende Nut im benachbarten Blendenelement (107, 108) eingreift.

## Claims

1. A contour collimator (1) for radiotherapy comprising a plural number of plate-shaped diaphragm elements (101, 102, 103, ...) movably arranged with respect to each other in a guide block (10) to form a contour diaphragm for a radiation beam (13) emitted by a radiation source (12) towards the collimator, and at least one drive for moving the diaphragm elements, characterised in that, a drive of its own (111, 112, 113, ...) is associated with each diaphragm element (101, 102, 103, ...);
that the drives (111, 112, 113, ...) of a group of diaphragm elements (101, 102, 103, ...) are essentially adjacent to each other; and
that a drive transmission of its own (121, 122, 123, ...) is arranged between each drive (111, 112, 113, ...) and the associated diaphragm element (101, 102, 103, ...).

2. A contour collimator according to claim 1, characterised in that the drives (111, 112, 113, ...) are essentially arranged in a semicircle.

3. A contour collimator according to claim 1 or 2, characterised in that each drive transmission (121, 122, 123, ...) comprises a flexible load-transmission element (131, 132, 133, ...) which is however rigid against tension and pressure, one end of which said load-transmission element is connected to the associated diaphragm element (101, 102, 103, ...) and the other end is connected to the associated drive (111, 112, 113, ...); said load transmission element being retained in a thrust guide (141, 142, 143, ...) so as to be movable in a translatory way.

4. A contour collimator according to claim 3, characterised in that each load transmission element (131, 132, 133, ...) is disconnectably coupled to the associated diaphragm element (101, 102, 103, ...) via a coupling (151, 152, 153, ...).

5. A contour collimator according to claim 3 or 4, characterised in that each load transmission element (131, 132, 133, ...) is disconnectably coupled to the associated drive (111, 112, 113, ...) via a further coupling.

6. A contour collimator according to one of claims 3 to 5, characterised in that each load transmission element (131, 132, 133, ...) comprises a spring band.

7. A contour collimator according to one of the preceding claims, characterised in that each drive (111, 112, 113, ...) is formed by a motor with linear action.

8. A contour collimator according to claim 7, characterised in that the motor (111, 112, 113, ...) is an electrical linear motor.

9. A contour collimator according to claim 7, characterised in that the motor (111, 112, 113, ...) is an electric motor with a linear-action mechanism, preferably a rack and pinion mechanism or a spindle mechanism.

10. A contour collimator according to one of the preceding claims, characterised in that the guide block (10) comprises an upper guide plate (16) and a lower guide plate (17) each comprising a plural number of upper guide grooves (161, 162, 163, ...) or lower guide grooves (171, 172, 173, ...) for the diaphragm elements (101, 102, 103, ...).

11. A contour collimator according to claim 10, characterised in that the upper guide plate (16) and the lower guide plate (17) each comprise a preferably rectangular aperture (18, 19), which apertures determine the maximum diaphragm aperture; with each aperture (18, 19) comprising a common middle plane (20) essentially aligned at a right angle to the longitudinal direction of the guide grooves (161, 162, 163, ...; 171, 172, 173, ...).

12. A contour collimator according to one of claims 3 to 11, characterised in that, the thrust guides (141, 142, 143, ...) are essentially arranged side by side in a thrust guide block (14) comprising thrust guide gaps diverging in fan-like curvature; with a load transmission element (131, 132, 133 ...) being accommodated in each of said thrust guide gaps.

13. A contour collimator according to one of the preceding claims, characterised in that each thrust guide block (14) is associated with two superimposed planes of drive arrangements, with each pair of superimposed drives (111, 112, 113, ...) acting on a load transmission element (131, 132, 133, ...) accommodated in adjacent thrust guides (141, 142, 143, ...).

14. A contour collimator according to one of the preceding claims, characterised in that two opposite groups of diaphragm elements (101, 102, 103, ...; 101', 102', 103', ...) movable in a translatory way, are provided, with every two opposite diaphragm elements (101, 101'; 102, 102'; 103, 103'; ...) being guided in a lower common guide groove (161, 161'; 162, 162'; 163, 163'; ...) and in an upper common guide groove (171, 171'; 172, 172'; 173, 173'; ...).

15. A contour collimator according to one of the preceding claims, characterised in that each diaphragm element (101, 101'; 102, 102'; 103, 103', ...) of a pair of opposite diaphragm elements with its free edge pointing away from the respective drive (111, 111', 112, 112', 113, 113', ...) is movable further than to the common middle plane (20) of the apertures (18, 19) in the upper guide plate (16) and in the lower guide plate (17).

16. A contour collimator according to one of the preceding claims, characterised in that each drive (111, 112, 113, ...) is associated with at least one displacement transducer (181, 182, 183, ...), preferably a potentiometer for acquiring the position of the respective diaphragm element (101, 102, 103, ...).

17. A contour collimator according to claim 16, characterised in that the displacement transducer (181, 182, 183, ...) comprises a sliding potentiometer activatable in a translatory way.

18. A contour collimator according to one of the preceding claims, characterised in that at least one of the diaphragm elements (106, 107, 108) located in the region of the central middle beam of the ray cluster (13) comprises at least one widening rib (23, 23', 24, 24') aligned in the direction of translation.

19. A contour collimator according to claim 18, characterised in that each widening rib (23, 23'; 24, 24') interacts with a corresponding groove in the adjacent diaphragm element (107, 108).

## Revendications

1. Collimateur profilé (1) pour la radiothérapie équipé d'une pluralité d'éléments de diaphragme (101, 102, 103, ...) prévus dans un bloc de guidage (10), disposés de façon à pouvoir se déplacer les uns par rapport aux autres et en forme de plaques, pour former un diaphragme profilé pour un faisceau de rayons (13) partant d'une source de rayonnement et axé sur le collimateur et d'au moins un organe d'entraînement pour le déplacement des éléments de diaphragme, caractérisé en ce qu'un organe d'entraînement (111, 112, 113, ...) propre est attribué à chaque élément de diaphragme (101, 102, 103, ...), en ce que les organes d'entraînement (111, 112, 113, ...) d'un groupe d'éléments de diaphragme (101, 102, 103, ...) sont disposés essentiellement les uns à côté des autres et en ce qu'un dispositif de transmission d'entraînement (121, 122, 123, ...) propre est prévu entre chaque organe d'entraînement (111, 112, 113, ...) et l'élément de diaphragme (101, 102, 103, ...) attribué.

2. Collimateur profilé selon la revendication 1, caractérisé en ce que les organes d'entraînement (111, 112, 113, ...) sont disposés principalement en forme de demi-cercle.

3. Collimateur profilé selon la revendication 1 ou 2, caractérisé en ce que chaque dispositif de transmission d'entraînement (121, 122, 123, ...) présente un élément de transmission de force (131, 132, 133, ...) flexible mais résistant à la traction et à la pression, qui est relié par l'une de ses extrémités à l'élément de diaphragme (101, 102, 103, ...) attribué et par son autre extrémité à l'organe d'entraînement (111, 112, 113, ...) attribué et est logé de façon à se déplacer par translation dans un guide coulissant (141, 142, 143, ...).

4. Collimateur profilé selon la revendication 3, caractérisé en ce que chaque élément de transmission de force (131, 132, 133, ...) est couplé de façon amovible par un assemblage de couplage (151, 152, 153, ...) à l'élément de diaphragme (101, 102, 103, ...) qui lui est attribué.

5. Collimateur profilé selon la revendication 3 ou 4, caractérisé en ce que chaque élément de transmission de force (131, 132, 133, ...) est relié de façon amovible par un autre assemblage de couplage à l'organe d'entraînement (111, 112, 113, ...) qui lui est attribué.

6. Collimateur profilé selon l'une quelconque des revendications 3 à 5, caractérisé en ce que chaque élément de transmission de force (131, 132, 133, ...) présente une bande à ressort.

7. Collimateur profilé selon l'une quelconque des revendications précédentes, caractérisé en ce que chaque organe d'entraînement (111, 112, 113, ...) est formé par un moteur agissant de façon linéaire.

8. Collimateur profilé selon la revendication 7, caractérisé en ce que le moteur (111, 112, 113, ...) est un moteur linéaire électrique.

9. Collimateur profilé selon la revendication 7, caractérisé en ce que le moteur (111, 112, 113, ...) est un moteur électrique avec un engrenage à effet linéaire, de préférence un engrenage à crémaillère ou un engrenage à tige.

10. Collimateur profilé selon l'une quelconque des revendications précédentes, caractérisé en ce que le bloc de guidage (10) présente une plaque de guidage supérieure (16) et une plaque de guidage inférieure (17), qui sont pourvues respectivement d'une pluralité de rainures de guidage supérieures (161, 162, 163, ...) et inférieures (171, 172, 173, ...) pour les éléments de diaphragme (101, 102, 103, ...).

11. Collimateur profilé selon la revendication 10, caractérisé en ce que la plaque de guidage supérieure (16) et la plaque de guidage inférieure (17) sont pourvues respectivement d'une ouverture (18, 19) de préférence rectangulaire, lesquelles ouvertures déterminent l'ouverture de diaphragme maximale et présentent un plan médian (20) commun et sensiblement perpendiculaire à la direction longitudinale des rainures de guidage (161, 162, 163, ...; 171, 172, 173, ...).

12. Collimateur profilé selon l'une quelconque des revendications 3 à 11, caractérisé en ce que les guides coulissants (141, 142, 143, ...) sont disposés sensiblement les uns à côté des autres dans un bloc de guidage coulissant (14) et présentent des fentes pour guide coulissant qui se séparent de façon courbée à la façon d'un éventail, dans lesquelles respectivement un élément de transmission de force (131, 132, 133, ...) est réceptionné et peut se déplacer par translation.

13. Collimateur profilé selon l'une quelconque des revendications précédentes, caractérisé en ce que deux plans superposés d'agencements avec organe d'entraînement sont attribués à chaque bloc de guidage coulissant (14), deux organes d'entraînement (111, 112, 113, ...) superposés sollicitant respectivement un élément de transmission de force (131, 132, 133, ...) réceptionné dans des guides coulissants (141, 142, 143, ...) juxtaposés.

14. Collimateur profilé selon l'une quelconque des revendications précédentes, caractérisé en ce qu'il est prévu dans le bloc de guidage (10) deux groupes situés en regard d'éléments de diaphragme (101, 102, 103, ...; 101', 102', 103', ...) pouvant être entraînés par translation, respectivement deux éléments de diaphragme (101, 101'; 102, 102'; 103, 103'; ...) disposés l'un en regard de l'autre étant guidés dans une rainure de guidage commune inférieure (161, 161'; 162, 162'; 163, 163'; ...) et une rainure de guidage commune supérieure (171, 171'; 172, 172'; 173, 173'; ...).

15. Collimateur profilé selon l'une quelconque des revendications précédentes, caractérisé en ce que chaque élément de diaphragme (101, 101', 102, 102', 103, 103', ...) d'une paire d'éléments de diaphragme situés l'un en face de l'autre avec son arête libre et opposée à l'organe d'entraînement respectif (111, 111', 112, 112', 113, 113', ...) peut être déplacé jusqu'au-delà du plan médian (20) commun des ouvertures (18, 19) dans les plaques de guidage supérieure (16) et inférieure (17).

16. Collimateur profilé selon l'une quelconque des revendications précédentes, caractérisé en ce qu'à chaque organe d'entraînement (111, 112, 113, ...) est attribué au moins un capteur de déplacement (181, 182, 183, ...) de préférence un potentiomètre pour l'enregistrement de la position de l'élément de diaphragme correspondant (101, 102, 103, ...)

17. Collimateur profilé selon la revendication 16, caractérisé en ce que le capteur de déplacement (181, 182, 183, ...) présente un potentiomètre coulissant actionnable par translation.

18. Collimateur profilé selon l'une quelconque des revendications précédentes, caractérisé en ce qu'au moins l'un des éléments de diaphragme (106, 107, 108) situés dans la zone du rayon central du faisceau de rayons (13) est pourvu d'au moins une nervure d'épaississement (23, 23', 24, 24') allant dans le sens de la translation.

19. Collimateur profilé selon la revendication 18, caractérisé en ce que chaque nervure d'épaississement (23, 23'; 24, 24') s'engage dans une gorge appropriée dans l'élément de diaphragme voisin (107, 108).
